# DEMANDE DE BREVET EUROPEEN

(11) **EP 2 196 191 A1**
(43) Date de publication de la demande: **16.06.2010**
(21) Numéro de dépôt: 09177539.5
(22) Date de dépôt: 30.11.2009
(51) Int. Cl.: A61K 8/49, A61Q 5/04, A61K 8/36

(54) **Composition cosmetique comprenant un compose heterocyclique disulfure et un agent reducteur et procede pour la deformation permanente des fibres keratiniques**

(30) Priorité: 11.12.2008 FR 0858473; 11.12.2008 FR 0858465; 11.12.2008 FR 0858475
(71) Demandeur: L'Oréal, 75008 Paris (FR)
(72) Inventeur: De Boni, Maxime, Tokyo 162-0842 (JP); Takahashi, Hiroshi, Tokyo 206-0802 (JP); Hirasawa, Yosuke, Yokohama-shi 225-0012 (JP)
(74) Mandataire: Fevrier, Murielle Françoise E.

(57) **Abrégé**

La présente invention a pour objet l'utilisation en tant qu'agent de traitement des fibres kératiniques particulièrement pour la déformation permanente des fibres kératiniques, i) d'au moins un composé hétérocyclique de formule (I) en présence de ii) au moins un agent réducteur et/ou, iii) de chaleur apportée par chauffage à une température comprise entre 50 et 250 °C, et/ou iv) à pH alcalin, lorsque iv) est présent pour diminuer la dégradation des fibres kératiniques. Elle a également pour objet un composition cosmétique comprenant i) au moins un composé hétérocyclique disulfure particulier et/ou i) au moins un agent réducteur et/ou ii) à pH alcalin, et un procédé de déformation permanente des fibres kératiniques et/ou pour diminuer la dégradation des fibres kératiniques en particulier des fibres kératiniques humaines tels que les cheveux. 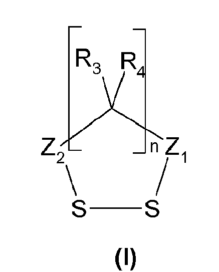 avec Z₁, Z₂, R₃, R₄ et n tels que définis dans la description.

Les procédés selon l'invention présentent l'avantage de transformer les fibres kératiniques de façon esthétique et durable face aux diverses agressions extérieures tout en gardant une cosmétique de fibre très convenable. Ils permettent d'obtenir des frisures et tenue de boucles plus performantes que celui des méthodes classiques n'utilisant pas de composés disulfures.

## Description

La présente invention a pour objet l'utilisation en tant qu'agent de traitement des fibres kératiniques particulièrement pour la déformation permanente des fibres kératiniques, i) d'au moins un composé hétérocyclique particulier en présence de ii) au moins un agent réducteur et/ou, iii) de chaleur apportée par chauffage à une température comprise entre 50 et 250 °C, et/ou iv) à pH alcalin, lorsque iv) est présent pour diminuer la dégradation des fibres kératiniques. Elle a également pour objet une composition cosmétique comprenant i) au moins un composé hétérocyclique disulfure particulier et/ou i) au moins un agent réducteur et/ou ii) à pH alcalin, et un procédé de déformation permanente des fibres kératiniques et/ou pour diminuer la dégradation des fibres kératiniques en particulier des fibres kératiniques humaines tels que les cheveux.

On sait que la technique la plus usuelle pour obtenir une déformation permanente des cheveux consiste, dans un premier temps, à réaliser l'ouverture des liaisons disulfures -S-S- de la kératine (cystine) en appliquant sur les cheveux préalablement mis sous tension (bigoudis et autres) une composition contenant un agent réducteur (étape de réduction) puis, après avoir rincé la chevelure ainsi traitée, à reconstituer dans un second temps lesdites liaisons disulfures en appliquant sur les cheveux toujours sous tension une composition oxydante (étape d'oxydation, dite aussi de fixation) de façon à donner finalement aux cheveux la forme recherchée appelée forme artificielle. Cette technique permet ainsi de réaliser indifféremment soit l'ondulation des cheveux, soit leur défrisage ou leur décrêpage.

La tenue de la forme artificielle et la dégradation des fibres kératiniques sont deux problématiques majeures dans la mise au point de produits de transformation desdites fibres, en particulier pour ceux utilisant des traitements réducteurs dans les permanentes ou les lissages. En effet, les deux étapes de réduction et d'oxydation mises en oeuvre dans les procédés classiques de déformation permanente, peuvent générer, lorsqu'elles sont répétées, des dégradations des fibres kératiniques. De plus les résultats de tenue de forme artificielle obtenus après mise en oeuvre de ces procédés sont souvent insuffisants pour l'utilisateur. Un autre moyen pour ouvrir les liaisons disulfures de la kératine ainsi que les composés hétérocycliques disulfures pour permanenter les cheveux est d'utiliser des radiations électromagnétiques (US 5,858,179). Cette technique nécessite au préalable l'utilisation d'une source électromagnétique pour activer les composés hétérocycliques disulfures.

Par ailleurs certaines méthodes visent à palier le problème de l'odeur de la permanente en utilisant des composés disulfures EP 1 129 687. En revanche ce document ne vise pas à améliorer la tenue de la forme des mèches.

Un autre document mentionne un procédé pour permanenter les cheveux mettant en oeuvre une composition comprenant de l'acide lipoïque et un agent réducteur (WO 2008/110223).

Cependant les permanentes obtenues avec ce procédé ne sont pas satisfaisantes en termes d'efficacité en particulier au niveau de la tenue et/ou en terme de dégradation des fibres.

Par ailleurs les technologies actuelles de coloration font appel très majoritairement à de la coloration d'oxydation. Ce procédé de coloration fait intervenir :
- des bases d'oxydation tels que des ortho ou paraphénylènediamines, des ortho ou paraaminophénols et des composés hétérocycliques, et
- des coupleurs tels que les métadiamines aromatiques, les métaaminophénols, les métadiphénols et certains composés hétérocycliques tels que des composés indoliques.

Les bases d'oxydation et coupleurs sont appelés colorants d'oxydation.

Les bases d'oxydations et les coupleurs sont capables de réagir ensemble en milieu oxydant pour générer des espèces polymérisées colorées.

Dans le même temps, les conditions alcalines du milieu oxydant utilisées dans la coloration d'oxydation permet :
- de faire gonfler les écailles des fibres kératiniques pour faciliter l'entrée des bases et des coupleurs à l'intérieur de la fibre, et
- de solubiliser la mélanine permettant d'éclaircir la fibre de 1 à 4 tons.

Ce dernier phénomène peut altérer l'intégrité de la fibre, notamment lors de traitement répétés avec ces compositions alcalines oxydantes utilisées seules ou en combinaison avec d'autres traitements chimiques tels que la permanente, la décoloration ou le défrisage alcalin.

Il a déjà été mentionné l'utilisation de l'acide α-lipoïque pour notamment réduire les dommages oxydatifs des cheveux, diminuer l'irritation du cuir chevelu et apaiser la sensibilité du cuir chevelu (WO 2007/140856). Cependant, les résultats obtenus avec l'acide α-lipoïque ne sont pas satisfaisant particulièrement en termes de dégradation des fibres traitées avec les agents oxydants. Par ailleurs on peut noter un changement de couleur, de nuance et de puissance tinctoriale en employant les méthodes de traitement de l'état de la technique.

Il existe donc un besoin de mettre à disposition des compositions cosmétiques permettant de diminuer la dégradation des fibres kératiniques, lors du traitement oxydatif des fibres kératiniques, tel que la coloration éclaircissante ou la décoloration, tout en évitant un changement notable de la couleur des fibres ainsi traitées, notamment au niveau de la nuance et de la puissance. Il existe également un besoin de mettre à disposition des compositions cosmétiques permettant la mise en forme artificielle des fibres kératiniques, facile à mettre en oeuvre, durable, ne présentant pas les inconvénients de dégradation accentuée des fibres kératiniques par application répétées, de la faible tenue dans le temps et du manque de cosmétique des fibres kératiniques après traitement.

Ces buts sont atteints par la présente invention qui a pour objet l'utilisation en tant qu'agent de traitement des fibres kératiniques, tels que les cheveux :
i) d'un ou plusieurs composé(s) hétérocyclique(s) disulfure(s) de formule **(I) :** 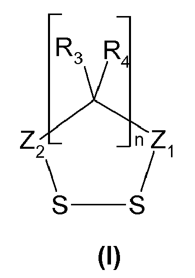 leurs sels avec un acide ou une base, leurs stéréoisomères, et leurs solvates tels que les hydrates,
   formule **(I)** dans laquelle :
   ■ **Z₁** et **Z₂,** identiques ou différents, représentent un groupe carbonyle ou une groupe divalent CR₁R₂ ou CR₅R₆;
   ■ **R₁** à **R₆,** identiques ou différents, représentent un atome d'hydrogène, ou un groupement choisi parmi : i) (C₁-C₈)alkyle, ii) aryle, iii) hydroxy, iv) (di)(C₁-C₈)(alkyl)amino, v) (C₁-C₈)alkoxy, vi) (poly)hydroxy(C₁-C₈)alkyle, vii) (di)(C₁-C₈)(alkyl)amino(C₁-C₈)alkyle, viii) carboxy, ix) carboxy(C₁-C₈)alkyle, x) (di)(C₁-C₈)(alkyl)aminocarbonyl(C₁-C₈)alkyle, xii) amino, xiii) aminocarbonyle(C₁-C₈)alkyle et xiv) (C₁-C₈)(alkyl)carbonyl(C₁-C₈)(alkyl)amino(C₁-C₈)alkyle ; ou alors
   ■ **R₁** et **R₃,** ou lorsque n vaut 2 ou 3 deux radicaux **R₃** portés par des atomes de carbone adjacents, forment ensemble avec les atomes de carbone qui les portent un groupe cycloalkyle, hétérocycloalkyle, aryle tel que phényle, ou hétéroaryle ; particulièrement phényle ;
   ■ **n** représente un entier égal à 1, 2 ou 3, particulièrement 1 ou 2 ;
      étant entendu que lorsque n vaut 2 ou 3 alors les groupement **R₃** et **R₄** peuvent être identiques ou différents entre eux ;
   le(s) dit(s) composé(s) hétérocyclique(s) disulfure(s) de formule **(I)** étant associé(s) à :
ii) un ou plusieurs agent(s) réducteur(s) et/ou,
iii) en présence de chaleur apportée par chauffage à une température comprise entre 50 et 250 °C, et/ou
iv) à pH alcalin,
étant entendu que lorsque le composé ou les composé(s) hétérocyclique(s) disulfure(s) de formule **(I)** est l'acide lipoïque et qu'il est associés à un ou plusieurs agent(s) réducteur(s), alors le dit acide lipoïque ne se trouve pas ensemble dans la même composition avec le ou les agent(s) réducteur(s).

Un autre objet de l'invention est une composition dans un support cosmétique comprenant :
i) un ou plusieurs composé(s) hétérocyclique(s) disulfure(s) de formule **(I')** 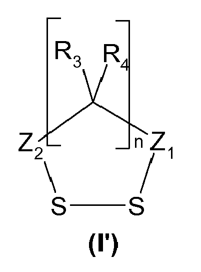 leurs sels avec un acide ou une base, leurs stéréoisomères, et leurs solvates tels que les hydrates,
   formule **(I')** dans laquelle :
   ■ **Z₁** et **Z₂,** identiques ou différents, représentent un groupe carbonyle ou une groupe divalent CR₁R₂ ou CR₅R₆ ;
   ■ **R₁, R₂, R₅** et **R₆,** identiques ou différents, représentent un atome d'hydrogène, ou un groupement choisi parmi : i) (C₁-C₈)alkyle, ii) aryle, iii) hydroxy, iv) (di)(C₁-C₈)(alkyl)amino, v) (C₁-C₈)alkoxy, vi) (poly)hydroxy(C₁-C₈)alkyle, vii) (di)(C₁-C₈)(alkyl)amino(C₁-C₈)alkyle, viii) carboxy, ix) carboxy(C₁-C₃)alkyle, x) (di)(C₁-C₈)(alkyl)aminocarbonyl(C₁-C₈)alkyle, et xii) (C₁-C₈)(alkyl)carbonyl(C₁-C₈)(alkyl)amino(C₁-C₈)alkyle ;
   ■ **R₃** et **R₄,** identiques ou différents, représentent un atome d'hydrogène, ou un groupement choisi parmi : i) (C₁-C₈)alkyle, ii) aryle, iii) hydroxy, iv) (di)(C₁-C₈)(alkyl)amino, v) (C₁-C₈)alkoxy, vi) (poly)hydroxy(C₁-C₈)alkyle, vii) (di)(C₁-C₈)(alkyl)amino(C₁-C₈)alkyle, viii) carboxy, ix) carboxy(C₁-C₈)alkyle, x) (di)(C₁-C₈)(alkyl)aminocarbonyl(C₁-C₈)alkyle, et xii) (C₁-C₈)(alkyl)carbonyl(C₁-C₈)(alkyl)amino(C₁-C₈)alkyle ; ou alors
   ■ **R₁** et **R₃,** ou lorsque n vaut 2 ou 3 deux radicaux **R₃** portés par des atomes de carbone adjacents, forment ensemble avec les atomes de carbone qui les portent un groupe cycloalkyle, hétérocycloalkyle, aryle tel que phényle, ou hétéroaryle ; particulièrement phényle ;
   ■ n représente un entier égal à 1, 2 ou 3, particulièrement 1 ou 2 ;
   étant entendu que lorsque n vaut 2 ou 3 alors les groupement **R₃** et **R₄** peuvent être identiques ou différents entre eux ; et
ii) un ou plusieurs agent(s) réducteur(s).

Un autre objet de l'invention est une composition à pH alcalin dans un support cosmétique comprenant un ou plusieurs composé(s) hétérocyclique(s) disulfure(s) de formule **(I)** ou **(I')** tel(s) que définis précédemment.

Un autre objet de l'invention concerne un procédé de traitement des fibres kératiniques mettant en oeuvre:
i) un ou plusieurs composé(s) de formule **(I)** ou **(I')** tel(s) que définis précédemment et
ii) un ou plusieurs agent(s) réducteur(s) ; et/ou
iii) à pH alcalin ; et/ou
iv) en présence de chaleur.

De tels procédés sont particulièrement simples à mettre en oeuvre et rapide. Contrairement à l'état de la technique, les composés hétérocycliques disulfures répondants à la formule **(I)** et **(I')** tels que définis précédemment ne nécessitent pas par exemple au préalable de subir l'effet d'une source électromagnétique pour agir dans le procédé de déformation permanente.

Les procédés selon l'invention sont particulièrement simples et rapides. Ils présentent en outre l'avantage de transformer les fibres kératiniques tels que les cheveux de façon esthétique et durable face aux diverses agressions extérieures tout en gardant une cosmétique de fibre très convenable. Notamment, les procédés selon l'invention permettent d'obtenir des frisures et tenue de boucles plus performantes que celui des méthodes classiques n'utilisant pas de composés disulfures.

L'utilisation de composés hétérocycliques disulfures de formule **(I)** et **(I')** tels que définis précédemment en milieu alcalin permet en outre de diminuer la dégradation des fibres kératiniques humaines tels que les cheveux sans changement notable dans la couleur des fibres.

D'autres caractéristiques, aspects et avantages de l'invention apparaîtront encore plus clairement à la lecture de la description détaillée qui va suivre, ainsi que des divers exemples concrets, mais nullement limitatifs, destinés à l'illustrer.

Bien que l'exposé qui suit s'articule essentiellement autour du cas particulier du traitement du cheveu, on notera ici que le procédé selon l'invention est applicable à toutes les fibres kératiniques. Les composés de formule **(I)** et **(I')** selon l'invention s'utilisent préférentiellement sur les fibres kératiniques humaines telles que les cheveux.

### Les composés hétérocycliques disulfures de formule (I) et (I') :

Un ou plusieurs composé(s) hétérocyclique(s) de formule **(I)** et **(I')** tels que définis précédemment sont utilisables selon l'invention, dans le procédé de traitement des fibres kératiniques et/ou sont contenus dans la composition cosmétique selon l'invention.

Ces composés hétérocycliques disulfures peuvent se présenter sous leur forme « réduites » (I)_{réd} i.e. sous leurs formes « ouvertes » correspondant à leurs formes dithiols suivante :

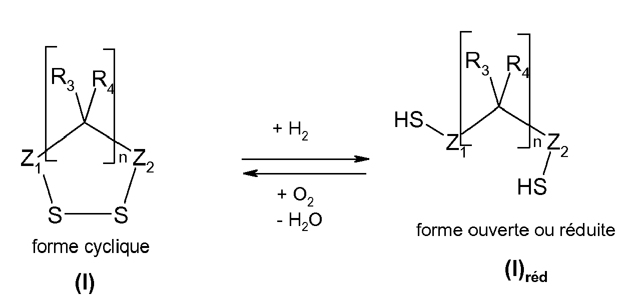

La forme réduite **(I)_{réd}** peut provenir de la réduction *in situ* du composé hétérocyclique disulfure **(I)** en présence d'agent réducteur tel que défini ci-dessous ou alors le composé **(I)_{réd}** est utilisé isolé sous forme réduite. Ce dernier peut alors se trouver dans la composition en présence d'un agent oxydant tel que défini ci-dessous, pour conduire également *in situ* à la forme cyclique **(I)** ou **(I').**

Particulièrement au moins un des radicaux R₁ à R₆ des composés de formule **(I)** ou **(I'),** est différent d'un atome d'hydrogène.

Selon un mode particulier de l'invention les composés de formule **(I)** ou **(I')** sont tels que **R₁** à **R₆**, identiques ou différents, représentent un atome d'hydrogène, ou un groupement choisi parmi : i) (C₁-C₈)alkyle, ii) aryle, iii) hydroxy, iv) (di)(C₁-C₈)(alkyl)amino, v) (C₁-C₈)alkoxy, vi) (poly)hydroxy(C₁-C₈)alkyle, vii) (di)(C₁-C₈)(alkyl)amino(C₁-C₈)alkyle, viii) carboxy, ix) amino, et x) (C₁-C₈)(alkyl)carbonyl(C₁-C₈)(alkyl)amino(C₁-C₈)alkyle.

Selon un mode particulier de l'invention les composés de formule **(I)** ou **(I')** sont tels que n vaut 1 ou 2. Une variante concerne les composés de formule **(I)** ou **(I')** dont n vaut 1. Selon une autre variante les composés sont de formule **(I)** ou **(I')** avec n valant 2.

Particulièrement les composés de formule **(I)** sont tels que R₁ à R₆ sont choisis parmi l'atome d'hydrogène, et les groupements hydroxy, carboxy, aminocarbonyl(C₁-C₃)alkyle.

Selon un autre mode de réalisation préféré de l'invention, les composés de formule **(I')** sont tels que R₁ à R₆ sont choisis parmi l'atome d'hydrogène, et les groupements hydroxy, carboxy.

Plus particulièrement les composés de formule **(I)** ou **(I')** sont tels que Z₁ représente CR₁R₂ et Z₂ représente CR₅R₆ avec R₁, R₂, R₅ et R₆ choisis parmi l'atome d'hydrogène, et les groupements hydroxy, carboxy et aminocarbonyl(C₁-C₃)alkyle. De préférence les composés de formule **(I)** ou **(I')** sont tels que R₃ et R₄, identiques ou différents, représentent un atome d'hydrogène ou un radical choisi parmi (C₁-C₃)alkyle, hydroxy et carboxy.

Une autre variante particulière de l'invention concerne des composés de formule **(I)** ou **(I')** dans lesquels Z₁ et_{/}ou Z₂ désignent un groupe carbonyle, et/ou R₁ et R₃ ou deux radicaux R₃ portés par des atomes de carbone adjacents forment ensemble avec les atomes de carbone qui les portent un cycle benzénique.

Etant entendu que dans la définition des radicaux R₁ à R₆ de la formule **(I)** ou **(I') :**
- Les radicaux alkyles sont des radicaux hydrocarbonés, saturés, linéaires ou ramifiés, généralement en C₁-C₆ particulièrement en C₁-C₄, tels que méthyle, éthyle, propyle, butyle, pentyle et hexyle.
- Les radicaux alcoxy sont des radicaux alkyle-oxy avec (C₁-C₆)alkyle de préférence en C₁-C₆ tels que méthoxy, éthoxy, propoxy et butoxy.
- Les radicaux (poly)hydroxyalkyles comportent sur le groupe (C₁-C₆)alkyle tel que défini précédemment, un ou plusieurs groupements hydroxyles substitués, particulier il s'agit de radical hydroxy(C₁-C₆) alkyle (C₁-C₆), tels que hydroxyméthyle, hydroxyéthyle et hydroxypropyle.
- Les radicaux cycloalkyles sont des radicaux cycloalkyles en C₄-C₈, pouvant contenir une insaturation, de préférence les radicaux cyclopentyle et cyclohexyle. Les radicaux cycloalkyles peuvent en outre être substitués, en particulier par des groupes alkyles, alcoxy, acide carboxylique, hydroxy, amine et cétone.
- Les radicaux hérérocycloalkyles sont des radicaux comprenant de 4 à 8 chainons, contenant de 1 à 3 hétéroatomes identiques ou différents, choisis parmi l'atome d'azote, d'oxygène et de soufre et contenant éventuellement de 1 à 3 atomes de carbone. Les radicaux hétérocycloalkyles peuvent en outre être substitués en particulier par les mêmes groupements que ceux définis pour les cycloalkyles.
- Les radicaux aryles sont des radicaux cycliques carbonés, mono ou polycycliques, comprenant de 6 à 14 atomes de carbone, et dont au moins un cycle est aromatique tel que le phényle, anthracényle, xanthényle, plus particulièrement phényle.
- Les radicaux hétéroaryles sont des radicaux cycliques, mono ou polycycliques, comprenant de 6 à 14 chainons, contenant de 1 à 3 hétéroatomes identiques ou différents, choisis parmi l'atome d'azote, d'oxygène et de soufre et contenant éventuellement de 1 à 3 atomes de carbone. Les radicaux hétéroaryles sont particulièrement choisis parmi les groupements suivants pyridyle, indolyle, furyle,
- Les radicaux hétéroaryles et aryles peuvent en outre être substitués en particulier par les mêmes groupements que ceux définis pour les cycloalkyles
- Les dérivés réduits sont les dérivés des composés hétérocycliques dithiols sous formes ouvertes de formule **(I)_{réd}** tels que définis précédemment.

A titre d'exemples de composés hétérocycliques disulfures utilisés dans le procédé selon l'invention qui fait intervenir les composés hétérocycliques de formule (I) on peut citer les composés (1) à (6), leurs stéréoisomères et leurs sels d'addition avec un acide ou une base ou leurs hydrates:

| **Dénomination** | **N° CAS** | **Formule chimique** |
|---|---|---|
| Acide lipoïque | 62-46-4 | 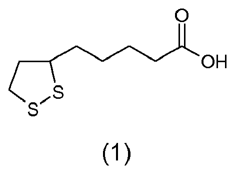 |
| Lipoamide | 940-69-2 | 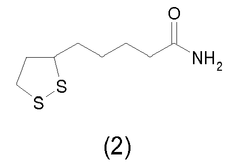 |
| Acide asparagusique | 2224-02-4 | 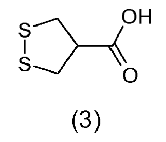 |
| 4,5-dihydroxy-1,2-dithiane | 14193-38-5 | 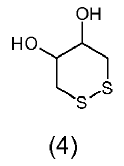 |
| 3H-1,2-benzodithiol-3-one | | 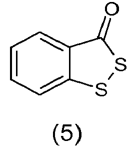 |
| Acide 1,2-dithiolane-4-méthyl-4- carboxylique | | 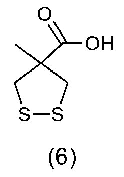 |

Selon un mode particulier de l'invention dans leur forme cyclique les composés hétérocycliques disulfures de formule **(I)** ou **(I')** présentent un logP inférieur à 3.

La valeur du logP représente le coefficient de partage du composé disulfure entre l'octanol et l'eau. Il peut être déterminé selon la méthode décrite dans l'article de Meylan et Howard « Atom / Fragment contribution method for estimating octanol-water partition coefficient », J. Pharm. Sci., 84, 83-92 (1995). Cette valeur peut également être calculée par des logiciels disponibles sur le marché qui déterminent la valeur de logP à partir de la structure de la molécule. A titre d'exemple on peut citer le logiciel Epiwin de l'agence environnementale des Etats-Unis.

Les composés hétérocycliques disulfures de formule **(I)** ou **(I')** ou leurs formes réduites mentionnés précédemment sont présents dans la composition cosmétique de l'invention à une concentration qui peut être comprise entre 1 et 20 %, de préférence entre 5 et 15 %, en poids par rapport au poids total de la composition les contenant.

A titre d'exemple de composés hétérocycliques disulfures de la composition selon l'invention comprenant au moins un agent réducteur et au moins un composé hétérocyclique de formule **(I')** tel que défini précédemment on peut citer les composés (3) à (6) tels que définis précédemment, leurs stéréoisomères, leurs sels d'addition avec un acide ou une base ou leurs hydrates.

L'acide intervenant dans les sels peut être minéral ou organique. A titre d'acides utilisables on peut citer l'acide chlorhydrique, l'acide sulfurique, l'acide phosphorique, l'acide acétique, l'acide tartrique, l'acide méthanesulfonique.

La base intervenant dans les sels peut être minérale ou organique. A titre de bases utilisables on peut citer les hydroxydes de sodium, de potassium ou d'ammonium, les carbonates ou bicarbonates de sodium, de potassium ou d'ammonium, les acides aminés basiques tels que l'arginine ou la lysine, les amines organiques non carboxyliques et en particulier les aminoalcools comme la monoéthanolamine ou la triéthanolamine.

Les composés hétérocycliques disulfures de formule **(I)** ou **(I')** mentionnés ci-avant sont présents dans une composition cosmétique à une concentration qui peut être comprise entre 1 et 20 %, de préférence entre 5 et 15 %, en poids par rapport au poids total de la composition les contenant.

### Les agents réducteurs :

Le procédé selon l'invention met également en oeuvre un ou plusieurs agent(s) réducteur(s).

Par agents réducteurs on entend un agent susceptible de réduire les fonctions disulfures de la kératine des fibres kératiniques. Pour ce faire, les thiols sont préférentiellement utilisés.

Plus particulièrement les thiols sont choisis parmi l'acide thioglycolique et ses sels, le monothioglycolate de glycérol ou de glycol, la cystéamine et ses dérivés acylés en C₁-C₄ tels que la N-acétyl cystéamine ou la N-propionyl cystéamine, la cystéine, la N-acétylcystéine, les esters de cystéine, tels que le cystéïnate de glycérol, les N-mercaptoalkylamides de sucres tels que le N-(mercapto-2-éthyl)gluconamide, l'acide thiolactique et ses esters tels que le monothiolactate de glycérol, l'acide 3-mercaptopropionique et ses esters, tels que le 3-mercaptopropionate de glycérol, l'acide thiomalique, l'acide 2-hydroxy 3-mercaptopropionique et ses esters, tels que le 2-hydroxy 3-mercaptopropionate de glycérol, la panthétéine, le thioglycérol, les N-(mercaptoalkyl)ω-hydroxyalkylamides décrits dans la demande de brevet EP-A-354 835 et les N-mono- ou N,N-dialkylmercapto 4-butyramides décrits dans la demande de brevet EP-A-368 763, les aminomercaptoalkylamides décrits dans la demande de brevet EP-A-432 000, les dérivés des acides N-(mercaptoalkyl) succinamiques ou des N-(mercaptoalkyl) succiminides décrits dans la demande de brevet EP-A-465 342, les alkylaminomercaptoalkylamides décrits dans la demande de brevet EP-A-514 282, le mélange de thioglycolate d'hydroxy-2-propyle et de thioglycolate d'hydroxy-2-méthyl-1 éthyle décrit dans la demande de brevet FR-A-2 679 448, les N-mercaptoalkyl alcanediamides décrits dans la demande de brevet EP-A-653 202.

On utilise de préférence l'acide thioglycolique, l'acide thiolactique, la cystéïne et ses divers dérivés, la cystéamine et ses dérivés, l'acide 3-mercaptopropionique, ainsi que leurs esters ou leurs sels, notamment le monothioglycolate de glycérol.

Ces agents actifs peuvent être utilisés seuls ou en mélange.

Les agents réducteurs ci-dessus sont en général présents en quantité comprise entre 0,01 et 20 % en poids par rapport au poids de la composition les comprenant, de préférence entre 0,1 et 10 % en poids par rapport au poids de la composition.

### Le pH alcalin :

Selon un mode de réalisation particulier de l'invention, la composition possède un pH alcalin, i.e. elle possède un pH supérieur à 7. Particulièrement le pH est peut aller de 8 à 14. Selon un mode préféré de l'invention le pH peut aller de 9 à 11.
Pour obtenir ce pH un ou plusieurs agent(s) alcalinisant(s) tel(s) que défini(s) ci-dessous sont ajoutés dans la composition selon l'invention comprenant un ou plusieurs composé(s) hétérocyclique(s) disulfure(s) de formule **(I)** ou **(I')** ou leurs formes réduites.

### La chaleur :

Selon un procédé de traitement des fibres kératiniques préféré de l'invention, le procédé est réalisé en présence de chaleur apportée par chauffage à une température comprise entre 50 °C et 250 °C Plus particulièrement entre 70 °C et 180 °C. Préférentiellement de 80 °C à 180°C.

Le traitement thermique par chauffage pendant tout ou partie du temps procédé peut être conduit par tout dispositif produisant de la chaleur et en particulier au moyen d'un casque de coiffure, d'un sèche-cheveux, d'un dispensateur de rayons infrarouges.

### Le procédé de traitement des fibres kératiniques :

Le procédé de traitement selon l'invention est réalisé en vue de le déformation permanente des fibres kératiniques tels que les cheveux lorsqu'il met en oeuvre le ou les composé(s) hétérocyclique(s) disulfure(s) de formule **(I)** ou **(I')** tels que définis précédemment avec un ou plusieurs agent(s) réducteur(s), et/ou en présence de chaleur. Lorsque le procédé met en oeuvre le ou les composé(s) hétérocyclique(s) disulfure(s) de formule **(I)** ou **(I')** tels que définis précédemment à pH alcalin pour alors il est réalisé en vue de diminuer la dégradation des fibres kératiniques. Etant entendu que lorsque i) à iv) sont mis en oeuvre dans le procédé, il peut alors être réalisé de la déformation permanente des fibres kératiniques tout en diminuant la dégradation desdites fibres.

Selon un procédé de traitement des fibres kératiniques préféré de l'invention, le procédé met en oeuvre dans un premier temps i) un ou plusieurs agent(s) réducteur(s) puis ii) un ou plusieurs composé(s) hétérocyclique(s) disulfure(s) de formule **(I)** tels que définis précédemment.

Selon un mode de réalisation particulier de l'invention le procédé comprend :
- une première étape dans laquelle on applique sur les fibres kératiniques à traiter un ou plusieurs agent(s) réducteur(s),
- une deuxième étape dans laquelle on rince lesdites fibres,
- une troisième étape dans laquelle on applique sur lesdites fibres un ou plusieurs composé(s) hétérocyclique(s) disulfure(s) de formule **(I)** tells que définis précédemment.

Un autre mode de réalisation particulier de l'invention consiste à appliquer sur des fibres kératiniques, une composition comprenant un ou plusieurs agent réducteurs tels que définis précédemment et un ou plusieurs composés hétérocycliques disulfures de formule **(I').** Plus particulièrement ce procédé selon l'invention comprend :
- une première étape dans laquelle on applique sur les fibres kératiniques à traiter une composition comprenant un ou plusieurs agent(s) réducteur(s), et un ou plusieurs composé(s) hétérocyclique(s) disulfure(s) de formule **(I')** tels que définis précédemment ; et
- une deuxième étape dans laquelle on rince lesdites fibres.

Il peut être utilisé dans le procédé des moyens de mise sous tension mécanique des fibres kératiniques. Ces moyens peuvent être mis en oeuvre avant ou pendant l'application d'un ou plusieurs agent(s) réducteur(s) ou avant ou pendant l'application de la composition comprenant un ou plusieurs agent(s) réducteur(s) et un ou plusieurs composé(s) hétérocyclique(s) disulfure(s) de formule **(I').** Après l'étape d'application du ou des composé(s) hétérocyclique(s) disulfure(s) de formule **(I)** ou de l'association du ou des agents réducteurs et le ou les composés hétérocycliques de formule **(I'),** les moyens de mise sous tension sont séparés des fibres kératiniques.

Selon un mode particulier du procédé de l'invention, le ou les agent(s) réducteur(s) sont appliqués sur des cheveux mouillés. Préférentiellement les cheveux mouillés ont été au préalable enroulés sur des moyens de tension tels que des rouleaux, préférentiellement ayant de 2 à 30 mm de diamètre. Le ou les agent(s) réducteur(s) ou l'association d' un ou plusieurs agents réducteur(s) et un ou plusieurs composé(s) hétérocyclique(s) disulfure(s) de formule **(I')** peuvent également être appliqués au fur et à mesure de l'enroulage des cheveux ou alors imprégnés dans le bigoudi lorsqu'il est par exemple en mousse.

On peut également appliquer, sur les cheveux enroulés, le ou les composé(s) hétérocyclique(s) disulfure(s) de formule **(I)** qui peuvent réagir alors avec les fibres kératiniques.

Selon une autre variante l'association du ou des agent(s) réducteur(s) et du ou des composé(s) hétérocyclique(s) disulfure(s) de formule **(I'),** est appliquée, sur les cheveux enroulés.

Généralement, on laisse ensuite agir le ou les agent(s) réducteur(s) pendant un temps de 5 à 60 minutes, de préférence de 15 à 45 minutes, puis on rince abondamment.

Selon un procédé particulier de l'invention, l'application du ou des composé(s) hétérocyclique(s) disulfure(s) de formule **(I)** ou de l'association d'un ou plusieurs agents réducteurs et un ou plusieurs composés hétérocycliques disulfures de formule **(I')** est suivie d'une étape de fixation des fibres kératiniques à l'aide d'un ou plusieurs agent(s) oxydant(s).

Eventuellement après l'étape d'application du ou des composé(s) hétérocyclique(s) disulfure(s) de formule **(I)** ou **(I')** et/ou l'étape d'application du ou des agent(s) oxydants, les fibres kératiniques sont rincées.

Le ou les agent(s) oxydant(s) permettent de reformer les liaisons disulfures de la kératine avec les composés de formule **(I)** ou **(I')** ayant réagit. Le temps de pose est préférentiellement de 2 à 10 minutes.

On peut également, après application du ou des composé(s) hétérocyclique(s) disulfure(s) de formule **(I)** ou **(I'),** soumettre la chevelure à un traitement thermique par chauffage à une température comprise entre 30 et 60°C pendant tout ou partie du temps de pose. Dans la pratique, cette opération peut être conduite au moyen d'un casque de coiffure, d'un sèche-cheveux, d'un dispensateur de rayons infrarouges et d'autres appareils chauffants classiques.

Lorsque l'on souhaite effectuer le défrisage ou le décrêpage au préalable des cheveux, i) on applique sur les cheveux un ou plusieurs agent(s) réducteur(s), puis l'on soumet les cheveux à une déformation mécanique par une opération de lissage des cheveux avec un peigne à larges dents, avec le dos d'un peigne ou à la main. Après généralement un temps de pose de 5 à 60 minutes, de préférence de 15 à 45 minutes, on procède à un nouveau lissage, puis on rince soigneusement et ii) on applique ensuite sur les fibres kératiniques mises sous tension ou non un ou plusieurs composé(s) hétérocyclique(s) disulfure(s) de formule **(I)** ou **(I'),** puis iii) on traite éventuellement les fibres kératiniques avec un ou plusieurs agent(s) oxydant(s), qu'on laisse agir pendant environ 2 à 10 minutes, puis on rince abondamment les cheveux.

Le procédé défini ci-dessus comprend une étape de séchage des fibres kératiniques. Le séchage des fibres kératiniques peut être un séchage partiel ou un séchage complet, et peut être réalisée par exemple avec un sèche-cheveux domestique ou encore un casque ou un bonnet chauffant, ou encore être effectuée grâce à un essorage soigneux. De préférence, le séchage des fibres kératiniques est un séchage complet. De préférence, on utilise un système chauffant pour sécher les cheveux.

Selon un mode préféré de l'invention, le procédé de traitement des fibres kératiniques selon l'invention est réalisé en présence de chaleur et met en oeuvre un ou plusieurs composé(s) hétérocyclique(s) disulfure(s) de formule **(I)** ou **(I')** tels que définis précédemment. La présence de chaleur peut être apportée à tous les stades du procédé et durant toute la mise en oeuvre du procédé.

Le procédé de l'invention s'adresse particulièrement à la déformation permanente de fibres kératiniques décolorées tels que les cheveux décolorés.

Particulièrement lors de la première étape du procédé selon l'invention les composés hétérocycliques disulfures de formule **(I)** ou **(I')** tels que définis précédemment sont appliqués sur les fibres kératiniques et dans une deuxième étape les fibres kératiniques sont chauffées à une température comprise entre 50 °C et 250 °C. Plus particulièrement à une température comprise entre 70 °C et 180 °C telle que 80 °C.

La durée de maintien sous chaleur est comprise entre 1 seconde et 4 heures, plus particulièrement de 10 minutes à 3 heures et préférentiellement de 10 minutes à 2 heures.

Selon un mode de réalisation particulier de l'invention le procédé met en oeuvre :
i) un ou plusieurs composé(s) hétérocyclique(s) disulfure(s) de formule **(I)** ou **(I')** tels que définis précédemment,
ii) un ou plusieurs agent(s) réducteur(s) ; et
iii) est réalisé en présence de chaleur préférentiellement à une température comprise entre 50 °C et 250 °C. Plus particulièrement à une température comprise entre 70 °C et 90 °C telle que 80 °C.

Selon un procédé particulier de l'invention, après application du ou des composé(s) hétérocyclique(s) disulfure(s) de formule **(I)** ou **(I'),** ou de l'association d'un ou plusieurs agents réducteur(s) et d'un ou plusieurs composé(s) hétérocyclique(s) disulfure(s) de formule **(I)** ou **(I'),** les fibres kératiniques sont chauffées.

Les fibres kératiniques peuvent être ensuite rincées et séchées.

Selon un mode de réalisation particulier le procédé de déformation permanente selon l'invention réalisé, met en oeuvre :
- une première étape dans laquelle on applique sur les fibres kératiniques à traiter un ou plusieurs agent(s) réducteur(s) tels que définis précédemment,
- une deuxième étape dans laquelle on rince lesdites fibres,
- une troisième étape dans laquelle on applique sur lesdites fibres un ou plusieurs composé(s) hétérocyclique(s) disulfure(s) de formule **(I)** ou **(I')** tels que définis précédemment et
- une quatrième étape où on chauffe lesdites fibres.

Selon un autre mode de réalisation particulier de l'invention le procédé comprend :
- une première étape dans laquelle on applique sur les fibres kératiniques à traiter une association d'un ou plusieurs agent(s) réducteur(s), et d'un ou plusieurs composé(s) hétérocyclique(s) disulfure(s) de formule **(I)** ou **(I')** tels que définis précédemment ;
- une deuxième étape dans laquelle on rince lesdites fibres et
- une troisième étape dans laquelle on chauffe les fibres kératiniques.

Il peut être utilisé dans le procédé des moyens de mise sous tension mécanique des fibres kératiniques.

Ces moyens peuvent être mis en oeuvre
a- avant ou pendant l'application du ou des composé(s) hétérocyclique(s) disulfure(s) de formule **(I)** ou **(I')** ou
b- avant ou pendant l'application de l'association d'un ou plusieurs agent(s) réducteur(s) et du ou des composé(s) hétérocyclique(s) disulfure(s) de formule **(I)** ou **(I')** ou
c- avant la présence de chaleur.
Les moyens de mise sous tension sont ensuite séparés des fibres kératiniques. Préférentiellement l'étape a ci-dessus est choisie.

Selon un mode particulier du procédé de l'invention, les cheveux peuvent être au préalable mouillés et sont enroulés sur des moyens de tension tels que des rouleaux. Lesdits rouleaux comportent préférentiellement un diamètre de 2 à 30 mm. Le ou les composé(s) hétérocyclique(s) disulfure(s) de formule **(I)** ou **(I')** ou l'association d'un ou plusieurs agents réducteur(s) et un ou plusieurs composé(s) hétérocyclique(s) disulfure(s) de formule **(I)** peuvent également être appliqués au fur et à mesure de l'enroulage des cheveux ou alors imprégnés dans les bigoudi lorsqu'ils sont par exemple en mousse.

Selon une autre variante le ou les composé(s) hétérocyclique(s) disulfure(s) de formule **(I)** ou l'association d' un ou plusieurs agents réducteur(s) et d'un ou plusieurs composé(s) hétérocyclique(s) disulfure(s) de formule **(I)** ou **(I')** peuvent être appliqués, sur les cheveux déjà enroulés.

Généralement, on laisse ensuite agir le ou les agent(s) réducteur(s) pendant un temps de 5 à 60 minutes, de préférence de 15 à 45 minutes, puis on rince abondamment.

Selon un procédé particulier de l'invention, l'application du ou des composé(s) hétérocyclique(s) disulfure(s) de formule **(I)** ou **(I')** est suivie d'une étape de fixation des fibres kératiniques à l'aide d'un ou plusieurs agent(s) oxydant(s).

Eventuellement après l'étape d'application du ou des composé(s) hétérocyclique(s) disulfure(s) de formule **(I)** ou **(I')** et/ou l'étape d'application du ou des agent(s) oxydants, les fibres kératiniques sont rincées.

Le ou les agent(s) oxydant(s) permettent de reformer les liaisons disulfures de la kératine avec les composés de formule **(I)** ou **(I')** ayant réagit. Le temps de pose est préférentiellement de 2 à 10 minutes.

Les fibres kératiniques peuvent être ensuite rincées et séchées.

Lorsque l'on souhaite effectuer le défrisage ou le décrêpage au préalable des cheveux, i) on applique sur les cheveux un ou plusieurs agent(s) réducteur(s), puis l'on soumet les cheveux à une déformation mécanique par une opération de lissage des cheveux avec un peigne à larges dents, avec le dos d'un peigne ou à la main. Après généralement un temps de pose de 5 à 60 minutes, de préférence de 15 à 45 minutes, on procède à un nouveau lissage, puis on rince soigneusement et ii) on applique ensuite sur les fibres kératiniques mises sous tension ou non un ou plusieurs composé(s) hétérocyclique(s) disulfure(s) de formule **(I)** ou **(I'),** on laisse pauser les composés de formule (I) pendant un temps de pause allant de 1 seconde à 4 heures plus particulièrement de 10 minutes à 3 heures et préférentiellement de 10 minutes à 2 heures sous chaleur puis iii) on traite éventuellement les fibres kératiniques avec un ou plusieurs agent(s) oxydant(s), qu'on laisse agir pendant environ 2 à 10 minutes, puis on rince abondamment les cheveux.

Selon un autre mode procédé de traitement des fibres kératiniques préféré de l'invention en vue de diminuer la dégradation desdites fibres, le procédé de traitement met en oeuvre à pH alcalin un ou plusieurs composés hétérocycliques disulfures de formule **(I)** ou **(I')** tel(s) que défini(s) précédemment ou leurs formes réduites. Le ou les composés hétérocycliques disulfures de formule **(I)** ou **(I')** peuvent se trouver dans une composition cosmétique ou être utilisés en poudre.

Selon un mode de réalisation particulier de l'invention le procédé comprend :
- une première étape dans laquelle on applique sur les fibres kératiniques à traiter une composition à pH alcalin prête à l'emploi comprenant un ou plusieurs composés hétérocycliques disulfures de formule **(I)** ou **(I')** ou leurs formes réduites tels que définis précédemment, et un ou plusieurs agents oxydants ; ladite composition ayant été préparée préférentiellement juste avant l'application par mélange extemporané d' un ou plusieurs composés hétérocycliques de formule **(I)** ou **(I')** ou de leurs formes réduites (tels quels ou présents dans une composition)et d'un ou plusieurs agents oxydants(tels quels ou présents dans une composition);
- une deuxième étape dans laquelle on laisse pendant un temps de pause de 1 minute à 2 heures la composition prête à l'emploi,
- une troisième étape dans laquelle les fibres kératiniques sont shampooinées, rincées et éventuellement séchées.

Selon un autre mode de réalisation particulier de l'invention le procédé comprend :
- une première étape dans laquelle on applique sur les fibres kératiniques à traiter un ou plusieurs composés hétérocycliques disulfures de formule **(I)** ou **(I')** ou leurs formes réduites éventuellement dans une composition le ou les comprenant et tels que définis précédemment, suivi éventuellement d'un temps de pause de 1 minute à 2 heures,
- dans une deuxième étape on applique un ou plusieurs agents oxydants éventuellement dans une composition le ou les comprenant, suivi éventuellement d'un temps de pause de 1 minute à 2 heures la composition,
- une troisième étape dans laquelle les fibres kératiniques sont shampooinées, rincés et éventuellement séchés.

Selon une variante de ces deux modes de réalisation le procédé selon l'invention met en oeuvre également des bases d'oxydation telles que définies précédemment et éventuellement des coupleurs tels que définis précédemment. Les bases et les coupleurs se trouvant préférentiellement dans la composition comprenant un ou plusieurs composés hétérocycliques disulfures de formule **(I)** ou **(I')** ou leurs formes réduites tels que définis précédemment.

Selon un mode particulier du procédé de l'invention, avant d'appliquer le ou les composés hétérocycliques disulfures de formule **(I)** ou **(I')** ou leurs formes réduites, les fibres kératiniques sont prétraitées par un ou plusieurs agents réducteurs tels que définis précédemment.

Généralement, on laisse ensuite agir le ou les agents réducteurs pendant un temps de 5 à 60 minutes, de préférence de 15 à 45 minutes, puis on rince abondamment.

Le procédé défini ci-dessus peut comprendre une étape de séchage des fibres kératiniques tel que défini précédemment.

Le ou les agent(s) réducteur(s), le ou les composé(s) hétérocyclique(s) disulfure(s) de formule **(I)** ou **(I')** et le ou les agent(s) oxydant(s) peuvent être contenus dans des compositions séparées ou dans une composition unique.

### Les compositions :

Les compositions selon l'invention comprennent un support cosmétique qui contient généralement de l'eau, un mélange d'eau et d'un ou plusieurs solvants organiques, ou un mélange de solvants organiques.

Par solvant organique, on entend une substance organique capable de dissoudre ou disperser une autre substance sans la modifier chimiquement.

### Les Solvants organiques:

A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, l'hexylène glycol, ainsi que les alcools aromatiques comme l'alcool benzylique ou le phénoxyéthanol.

Les solvants organiques sont présents dans des proportions de préférence comprises entre 1 et 40 % en poids environ par rapport au poids total de la composition tinctoriale, et encore plus préférentiellement entre 5 et 30 % en poids environ.

### Les Adjuvants:

La ou les compositions du procédé de coloration conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiant.

Lesdits adjuvants sont choisis de préférence parmi des agents tensioactifs tels que des tensioactifs anioniques, non ioniques ou leurs mélanges et des agents épaississants minéraux ou organiques.

Les adjuvants ci-dessus sont en général présents en quantité comprise pour chacun d'eux entre 0,01 et 40 % en poids par rapport au poids de la composition, de préférence entre 0,1 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition ou aux composition(s) utiles dans le procédé de déformation conforme à l'invention ne soient pas, ou substantiellement pas, altérées par la ou les adjonctions envisagées.

Les compositions utilisées selon l'invention et notamment celle comprenant un ou plusieurs composés hétérocycliques disulfures de formule **(I)** ou **(I')** peuvent comprendre en outre un ou plusieurs agents alcalinisants. Ces agents alcalinisants contribuent en particulier à ajuster le pH.

### Les agents alcalinisants :

L'agent alcalinisant utilisé dans les compositions utiles selon l'invention est un agent permettant d'augmenter le pH de la ou des compositions dans lequel il se trouve. L'agent alcalinisant est une base de Bronsted, de Lowry ou de Lewis. Il peut être minéral ou organique.

Particulièrement l'agent alcalinisant est choisi parmi :
a) l'ammoniaque,
b) les alcanolamines telles que les mono-, di- et triéthanolamines ainsi que leurs dérivés,
c) les éthylènediamines oxyéthylénées et/ou oxypropylénées,
d) les hydroxydes minéraux ou organiques,
e) les silicates de métaux alcalins tels que les métasilicates de sodium,
f) les acides aminés de préférences basiques comme l'arginine, la lysine, l'ornithine, la citruline et l'hystidine,
g) les (bi)carbonates particulièrement d'amine primaire, secondaire ou tertiaire (ammonium), de métal alcalin ou alcalino-terreux et
h) les composés de formule **(II)** suivante : 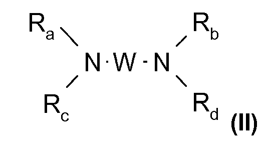
formule **(II)** dans laquelle W est un reste propylène éventuellement substitué par un groupement hydroxy ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

Les hydroxydes minéraux ou organiques sont de préférences choisis parmi a) les hydroxydes d'un métal alcalin, b) les hydroxydes d'un métal alcalino-terreux, comme les hydroxydes de sodium ou de potassium, c) les hydroxydes d'un métal de transition, tels que les hydroxydes des métaux des groupes III, IV, V et VI, d) les hydroxydes des lanthanides ou des actinides, les hydroxydes d'ammoniums quaternaires et l'hydroxyde de guanidinium.

L'hydroxyde peut être formé in situ comme par exemple l'hydroxyde de guanidine par réaction d'hydroxyde de calcium et de carbonate de guanidine.

Le ou les agent(s) alcalinisant(s) tels que définis précédemment représentent de préférence de 0,001% à 10 % en poids du poids de la composition les contenant. Plus particulièrement de 0,005 % à 8 % en poids de la composition.

Lorsqu'une composition dans l'invention comprend un ou plusieurs agents réducteurs le pH de la composition est de préférence compris entre 5 et 11,5 et encore plus préférentiellement entre 6 et 10.

Les compositions utiles dans l'invention peuvent se présenter sous la forme d'une lotion, épaissie ou non, d'une crème, d'un gel ou de toute autre forme appropriée et peut contenir des additifs connus pour leur utilisation dans les compositions réductrices pour la déformation permanente des cheveux.

Les compositions utiles dans l'invention peuvent être également du type exothermique, c'est-à-dire provoquant un certain échauffement lors de l'application sur les cheveux, ce qui apporte un agrément à la personne qui subit la permanente. Cette composition peut également contenir un solvant tel que par exemple de l'éthanol, du propanol, ou de l'isopropanol, du glycérol, du propylène glycol ou encore du diéthylène glycol à une concentration maximale de 20 % par rapport au poids total de la composition.

Bien entendu, l'homme de l'art veillera à choisir ce ou ces éventuels composés complémentaires de manière telle que les propriétés avantageuses attachées intrinsèquement à la composition ou aux compositions utiles dans le procédé de déformation conforme à l'invention ne soient pas, ou substantiellement pas, altérées par la ou les adjonctions envisagées.

Le pH des compositions peut être ajusté classiquement par ajout de bases ou agents alcalinisants tels que définis ci-dessus ou par ajout d'acides.

Les acides peuvent être minéraux ou organiques. Ils sont particulièrement choisis parmi l'acide chlorhydrique, l'acide orthophosphorique, l'acide sulfurique, les acides carboxyliques comme l'acide acétique, l'acide tartrique, l'acide citrique, l'acide lactique, les acides sulfoniques.

### Les agents oxydants:

De préférence le ou les composés hétérocycliques disulfures de formule **(I)** ou **(I')** ou leurs formes réduites sont mis en oeuvre en présence d'un ou plusieurs agent(s) oxydant(s) ou alors la composition selon l'invention à pH alcalin qui comprend un ou plusieurs composés hétérocycliques disulfures de formule **(I)** ou **(I'),** peut en outre contenir un ou plusieurs agents oxydants.

Les agents oxydants sont choisis de préférence parmi l'eau oxygénée ou peroxyde d'hydrogène, les bromates alcalins, les persels tels que les persulfates perborates ou percarbonates , ou les les polythionates et les mélanges de ces agents oxydants.

La concentration en peroxyde d'hydrogène peut varier de 0,1 à 20 %et de préférence de 0,1 à 10 %, la concentration en bromate alcalin de 2 à 12 % et celle en persel de 0,1 à 15 %, en poids par rapport au poids total de la composition oxydante.

Le ou les agent(s) oxydant(s) peuvent se trouver dans une composition oxydante. Les agents oxydants sont choisis de préférence parmi l'eau oxygénée ou peroxyde d'hydrogène, les bromates alcalins, les persels, les polythionates et un mélange de bromate alcalin et de persel. La concentration en eau oxygénée peut varier de 1 à 20 volumes et de préférence de 1 à 10, la concentration en bromate alcalin de 2 à 12 % et celle en persel de 0,1 à 15 %, en poids par rapport au poids total de la composition oxydante.

Le pH de la composition oxydante est généralement compris entre 2 et 10 et il est préférentiellement acide i.e. compris entre 2 et 5.

### Les colorants d'oxydation :

L'invention peut mettre en oeuvre une composition tinctoriale comprenant une ou plusieurs bases d'oxydation classiquement utilisées en teinture d'oxydation, ou alors la composition selon l'invention qui comprend un ou plusieurs composés hétérocycliques disulfures de formule (I), peut en outre contenir une ou plusieurs bases d'oxydation classiquement utilisées en teinture d'oxydation.

A titre d'exemple, ces bases d'oxydation sont choisies parmi les para-phénylènediamines, les bis-phénylalkylènediamines, les para-aminophénols, les bis-para-aminophénols, les ortho-aminophénols, les bases hétérocycliques et leurs sels d'addition.

Parmi les para-phénylènediamines, on peut citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylène-diamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylène-diamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

De préférence, on utilisera un 4,5-diaminopyrazole et encore plus préférentiellement le 4,5-diamino-1-(β-hydroxyéthyl)-pyrazole et/ou l'un des ses sels.

A titre de dérivés pyrazoliques, on peut également citer les diamino N,N-dihydropyrazolopyrazolones et notamment celles décrites dans la demande FR-A-2 886 136 telles que les composés suivants et leurs sels d'addition : 2,3-diamino-6,7-dihydro-1H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diéthyl-5-(pyrrolidin-1 -yl)-1,2-dihydro-pyrazol-3-one, 4-amino-5-(3-diméthylamino-pyrrolidin-1-yl)-1,2-diéthyl-1,2-dihydro-pyrazol-3-one, 2,3-diamino-6-hydroxy-6,7-dihydro-1H,5H-pyrazolo[1,2-a]pyrazol-1-one.

On préférera utiliser la 2,3-diamino-6,7-dihydro-1H,5H-pyrazolo[1,2-a]pyrazol-1-one et/ou un de ses sels.

A titre de bases hétérocycliques, on utilisera préférentiellement le 4,5-diamino-1-(β-hydroxyéthyl)pyrazole et/ou la 2,3-diamino-6,7-dihydro-1H,5H-pyrazolo[1,2-a]pyrazol-1-one et/ou un de leurs sels.

La ou les bases d'oxydation utilisables dans l'invention et notamment éventuellement présentes dans la composition comprenant un ou plusieurs composés hétérocycliques disulfures de formule **(I)** ou leurs formes réduites sont en général présentent chacune en quantité comprise entre 0,001 à 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

L'invention peut mettre en oeuvre une ou plusieurs bases d'oxydation telles que définis précédemment et en outre un ou plusieurs coupleur(s) conventionnellement utilisés en teinture d'oxydation ou alors la composition selon l'invention qui comprend un ou plusieurs composés hétérocycliques disulfures de formule **(I)** ou **(I')** ou leurs formes réduite, et une ou plusieurs base(s) d'oxydation peut contenir en outre un ou plusieurs coupleurs conventionnellement utilisés pour la teinture de fibres kératiniques.

Parmi ces coupleurs, on peut notamment citer les méta-phénylènediamines, les méta-aminophénols, les méta-diphénols, les coupleurs naphtaléniques, les coupleurs hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition selon la présente invention, le ou les coupleurs sont chacun généralement présents en quantité comprise entre 0,001 et 10 % en poids environ du poids total de la composition tinctoriale, de préférence entre 0,005 et 6 %.

### Les Colorants additionnels directs :

L'invention peut en outre mettre en oeuvre un ou plusieurs colorants additionnel(s) directs qui peuvent être présents dans la composition selon l'invention.

Ces colorants directs sont par exemple choisis parmi ceux classiquement utilisés en coloration directe, et parmi lesquels on peut citer tous les colorants aromatiques et/ou non aromatiques d'utilisation courante tels que les colorants directs nitrés benzéniques neutres, acides ou cationiques, les colorants directs azoïques neutres, acides ou cationiques, les colorants directs quinoniques et en particulier anthraquinoniques neutres, acides ou cationiques, les colorants directs aziniques, triarylméthaniques, indoaminiques, les méthines, les styriles, les porphyrines, métalloporphyrines, les phtalocyanines, les cyanines méthiniques, et les colorants fluorescents.

Parmi les colorants directs naturels, on peut citer la lawsone, la juglone, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Plus particulièrement les colorants directs sont choisis parmi le Basic Red 51, le Basic Orange 31, le Basic Yellow 87 et le Basic Red 22.

| | |
|---|---|
| 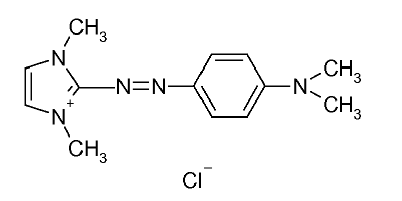 | 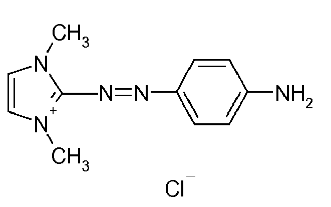 |
| Basic Red 51 | Basic Orange 31 |
| 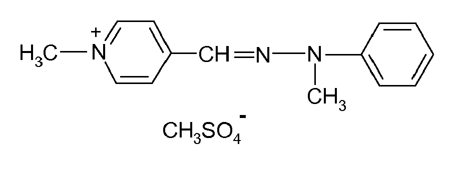 | 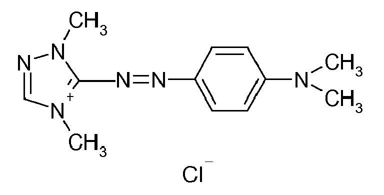 |
| Basic Yellow 87 | Basic Red 22 |

Les autres caractéristiques communes des compositions de l'invention décrites précédemment s'appliquent à cette composition oxydante.

### Les moyens de mise sous tension

L'étape de mise sous tension des fibres kératiniques peut être réalisée par tout moyen et par exemple avec des élastiques, des pinces, des peignes, des barrettes, des boudins ou encore avec des bigoudis cylindriques classiques ou rouleaux, et des bigoudis de type « tulipe ».

Les bigoudis peuvent être en mousse comme par exemple ceux décrits dans le document US-5,992,425. Dans ce cas le rouleau en mousse peut être imprégné d'un produit capillaire tel que la composition cosmétique comprenant au moins un agent réducteur ou ladite composition comprenant en outre au moins un composé hétérocyclique disulfure de formule **(I)** ou **(I')** tel que défini précédemment et sur lequel on enroule la mèche de fibres kératiniques dans le but d'enduire cette mèche de produit sur toute sa longueur d'enroulement. Des bigoudis « tulipe » se composent d'une tige allongée formant un corps terminé à une extrémité par une tête dotée d'au moins une perforation. Le corps du bigoudi est réalisé en matière souple et flexible, de sorte que son extrémité libre puisse être introduite dans la perforation de la tête et y être maintenue par serrage élastique. Un bigoudi du type défini ci-dessus est par exemple décrit dans la demande de brevet FR2602650. L'utilisation d'un bigoudi « tulipe» permet de contrôler la tension appliquée aux cheveux et permet ainsi de donner une forme arrondie aux mèches de cheveux pendant la durée du traitement.

Plus particulièrement les moyens de mises sous tension sont des bigoudis ayant un diamètre de 2 à 30 mm.

### EXEMPLES DE DEFORMATIONS PERMANENTES

### EXEMPLES 1 et 2

### Compositions

Les compositions suivantes ont été préparées (teneurs exprimées en grammes de matière active) :

### Composition 1 (lotion réductrice) :

| ingrédients | g (MA) |
|---|---|
| Acide thioglycolique | 7 |
| Ammoniaque (en solution aqueuse à 20% en ammoniac) | 9,1 |
| Bicarbonate d'ammonium | 2,5 |
| Chlorure d'ammonium | 1,7 |
| Acide diéthylène triamine pentaacétique (sel de sodium à 40% dans l'eau) | 0,4 |
| Eau déminéralisée | qsp 100 |

### Compositions 2 et 3 (intra-permanente) comprenant un composé hétérocyclique disulfure de formule (I) :

| ingrédients | Composition 2 g (MA) | Composition 3 g (MA) |
|---|---|---|
| Acide lipoïque | 0,5 | - |
| Trans-4,5-dihydroxy-1,2-dithiane | - | 10 |
| Monoéthanolamine | qsp pH 8 | qsp pH 6,5 |
| Eau déminéralisée | qsp 100 | qsp 100 |

### Composition 4 (fixateur) comprenant l'agent oxydant :

| ingrédients | g (MA) |
|---|---|
| Peroxyde d'hydrogène à 35 % dans l'eau | 5,15 |
| Salicylate de sodium | 0,01 |
| Sulfate de 8-hydroxyquinoléine | 0,0125 |
| Acide citrique | qsp pH 3 |
| Eau déminéralisée | qsp 100 |

### Procédé d'application

- Etape 1 :: une mèche de cheveux de 1 gramme est enroulée sur un bigoudi de 17 mm de diamètre,
- Etape 2 :: la composition réductrice **1** est appliquée dans un rapport de bain de 1,5 (1,5 gramme de formulation pour 1 gramme de cheveux) pendant 15 minutes,
- Etape 3 :: la mèche est rincée à l'eau claire,
- Etape 4 :: la composition **2** (ou **3)** est appliquée dans un rapport de bain de 1,5 pendant 10 minutes,
- Etape 5 :: la composition **4** est appliquée dans un rapport de bain de 1,5 pendant 5 minutes,
- Etape 6 :: la mèche de cheveux est déroulée, rincée à l'eau claire puis séchée.
Le traitement ne faisant pas intervenir l'étape 4 est choisi comme essai de référence.

### Exemple 1

Une mèche de cheveux naturels caucasiens (hauteur de ton de 3) et une mèche de cheveux décolorés (décoloration de 30 minutes à 35°C à l'aide d'une composition décolorante constituée d'un mélange poids pour poids de Platifiz Précision et d'eau oxygénée 20 volumes, références commerciales L'Oréal Professionnel) ont été permanentées selon le procédé indiqué ci-dessus incluant la composition 2.

Les mèches ainsi traitées ont été comparées aux mèches de référence (traitées sans l'étape d'intra-permanente) en termes de performances de frisure et de tenue de la boucle.

La composition et le procédé de l'invention permettent un résultat de boucle supérieur au procédé classique, que ce soit sur cheveux naturels que sur cheveux décolorés.

Une épreuve de tenue de la boucle est réalisée en maintenant les mèches sous tension pendant 5 heures à 40°C à 100% d'humidité relative. A l'issue du test de tenue, les mèches traitées selon le procédé de permanente de l'invention présentent des rendements de boucle supérieurs aux mèches de référence.

### Exemple 2

Une mèche de cheveux naturels caucasiens (hauteur de ton de 3) et une mèche de cheveux décolorés (cf. exemple 1) ont été permanentées selon le procédé indiqué ci-dessus incluant la composition 3.

Les mèches ainsi traitées ont été comparées aux mèches de référence (traitées sans l'étape d'intra-permanente) en termes de performances de frisure et de tenue de la boucle.

La composition et le procédé de l'invention offrent un résultat de boucle supérieur au procédé classique, que ce soit sur cheveux naturels que sur cheveux décolorés.

Les mèches ainsi permanentées ont subi l'épreuve de tenue de la boucle indiquée dans l'exemple 1. Les frisures obtenues avec le traitement de permanente de l'invention sont plus tenaces que les frisures obtenues selon le traitement de référence.

### Exemple 3 : comparatif avec une lotion réductrice comprenant l'acide lipoïque

La composition réductrice suivante a été préparée (teneurs exprimées en grammes de matière active) :

### Composition 5 (lotion réductrice comparative comprenant l'acide lipoïque) :

| ingrédients | g (MA) |
|---|---|
| Acide thioglycolique | 7 |
| Ammoniaque (en solution aqueuse à 20% en ammoniac) | 9,1 |
| Acide lipoïque | 0,5 |
| Bicarbonate d'ammonium | 2,5 |
| Chlorure d'ammonium | 1,7 |
| Acide diéthylène triamine pentaacétique (sel de sodium à 40% dans l'eau) | 0,4 |
| Eau déminéralisée | qsp 100 |

### Procédé d'application A selon l'invention :

Une mèche de 1 g de cheveux naturels caucasiens (hauteur de ton de 3) a été permanentée selon le procédé suivant :
- Etape 1 :: la mèche de cheveux est enroulée sur un bigoudi d'1,7 cm de diamètre,
- Etape 2 :: la lotion réductrice Composition **1** est appliquée dans un rapport de bain de 1,5 pendant 15 minutes,
- Etape 3 :: la mèche est rincée à l'eau claire,
- Etape 4 :: la Composition **2** est appliquée dans un rapport de bain de 1,5 pendant 10 minutes,
- Etape 5 :: le fixateur Composition **4** est appliqué dans un rapport de bain de 1,5 pendant 5 minutes,
- Etape 6 :: la mèche de cheveux est rincée à l'eau claire, déroulée, puis séchée.

### Résultat pour le procédé A selon l'invention :

La composition et le procédé selon l'invention offrent un très bon résultat de boucle en terme de frisure et d'homogénéité de la boucle. Le cheveu est par ailleurs très doux au toucher.

### Procédé d'application B mettant en oeuvre la composition 5 :

Une mèche de 1g de cheveux naturels caucasiens (hauteur de ton de 3) a été permanentée selon le procédé suivant :
- Etape 1 :: la mèche de cheveux est enroulée sur un bigoudi d'1,7 cm de diamètre,
- Etape 2 :: la composition réductrice 5 est appliquée dans un dans un rapport de bain de 1,5 (1,5 gramme de formulation pour 1 gramme de cheveux) pendant 15 minutes,
- Etape 3 :: la mèche est rincée à l'eau claire,
- Etape 4 :: la composition 4 est appliquée dans un rapport de bain de 1,5 pendant 5 minutes,
- Etape 5 :: la mèche de cheveux est rincée à l'eau claire, déroulée, puis séchée.

### Résultat pour le procédé d'application B comparatif :

La boucle est de qualité inférieure à celle obtenue par le procédé A selon l'invention en terme notamment de frisure (nervosité et d'homogénéité). Le toucher reste par ailleurs inchangé selon le procédé B sans noter d'amélioration au toucher (pas de douceur particulière) contrairement à ce qui a été observé avec le procédé A.

### Exemple 4 : Composition avec l'acide lipoïque et le lipoamide en milieu réducteur

### Composition 6 et 7 (lotions réductrices) :

| | Composition **6** | Composition **7** |
|---|---|---|
| Acide thioglycolique | 7 | 7 |
| Acide lipoïque | 0,1 | - |
| Lipoamide | - | 0,1 |
| Ammoniaque (en solution aqueuse à 20% en ammoniac) | 9,1 | 9,1 |
| Bicarbonate d'ammonium | 2,5 | 2,5 |
| Chlorure d'ammonium | 1,7 | 1,7 |
| Acide diéthylène triamine pentaacétique (sel de sodium à 40% dans l'eau) | 0,4 | 0,4 |
| Eau déminéralisée | qsp 100 | qsp 100 |

### Procédé d'application

### Une mèche de 1g de cheveux naturels japonais a été permanentée selon le procédé suivant :

Etape 1 : la mèche de cheveux est enroulée sur un bigoudi d'1,7 cm de diamètre
Etape 2 : la composition réductrice 6 ou 7 est appliquée dans un dans un rapport de bain de 1,5 pendant 15 minutes,
Etape 3 : la mèche est rincée à l'eau claire,
Etape 4 : la composition **4** est appliquée dans un rapport de bain de 1,5 pendant 5 minutes,
Etape 5 : la mèche de cheveux est rincée à l'eau claire, déroulée, puis séchée.

La frisure obtenue avec la composition **7** (lipoamide) est plus homogène et esthétique que celle obtenue avec la composition **6** (acide lipoïque).

### EXEMPLES 5 et 6

### Compositions

On a préparé les compositions suivantes (teneurs exprimées en grammes de matière active) :

| Ingrédients | Composition **3'** | Composition **4'** | Composition **5'** | Composition **6'** |
|---|---|---|---|---|
| Acide thioglycolique | 1 | - | - | - |
| Dithioglycolate de diammonium (en solution aq. à 48%) | - | 2,1 | - | - |
| Acide lipoïque | - | - | 10 | - |
| Trans-4,5-dihydroxy-1,2-dithiane | - | - | - | 3 |
| Ammoniaque (en solution aq. à 20% en ammoniac) | qsp pH 8,8 | Qs H 8,8 | qsp pH 8,8 | qsp pH 10 |
| Eau déminéralisée | qsp 100 | qsp 100 | qsp 100 | qsp 100 |

### Exemple 5

Sur des mèches de 1 g de cheveux naturels japonais et de cheveux décolorés (décoloration de 30 minutes à 35°C à l'aide d'une composition décolorante constituée d'un mélange poids pour poids de Platifiz Précision et d'eau oxygénée 20 volumes, références commerciales L'Oréal Professionnel) japonais enroulées sur des bigoudis de 17 mm, la composition 3' a été appliquée dans un rapport de bain de 2 pendant 2 heures à 80°C (appareil de Digital Perm, Oohiro ODIS-2) A l'issue du temps de pause, les mèches ont été rincées puis séchées.

Les mèches ainsi traitées ont été comparées à des mèches traitées avec les compositions 3' et 4' dans les mêmes conditions.

La composition 5' a permis de mettre en forme les cheveux de façon esthétique. Les mises en forme obtenues avec la composition 3' sont hétérogènes. Celles obtenues avec la composition 4' sont très faibles.

### Exemple 6

Sur des mèches de 1 g de cheveux naturels japonais et de cheveux décolorés (cf. traitement de décoloration de l'exemple 5) japonais enroulés sur des bigoudis de 17 mm, la composition 6' a été appliquée dans un rapport de bain de 2 pendant deux heures à 80°C (appareil de Digital Perm, Oohiro ODIS-2). A l'issue du temps de pause, les mèches ont été rincées puis séchées.

Les mèches ainsi traitées ont été comparées à des mèches traitées avec la composition 3' dans les mêmes conditions.

La composition 6' est plus efficace que la composition 1 en termes de performance de frisure. Les mises en forme obtenues avec la composition 1 sont hétérogènes.

Les mèches traitées avec les compositions 3' et 6' ont été immergées pendant 4 minutes dans une solution aqueuse à 40 °C contenant 8 % de lauryl éther sulfate de sodium. Les frisures obtenues avec la composition 6' sont plus tenaces et plus esthétiques que celles obtenues avec la composition 3'.

### Exemple 7

Sur mèches de 1 g de cheveux naturels japonais et de cheveux décolorés (cf. traitement de décoloration de l'exemple 5 ci-dessus) japonais enroulées sur des bigoudis de 1,7 cm, la composition 5' contenant 10% d'acide lipoïque a été appliquée dans un rapport de bain de 2 pendant 2 heures à 80°C. A l'issue du temps de pause, chaque mèche a été rincée puis séchée.

Les mèches ainsi traitées ont été comparées à des mèches traitées avec la même composition 5' pendant 2 heures à température ambiante.

De façon inattendue, il apparaît que les mèches de cheveux traités par la composition 5' en présence de chaleur présentent une boucle esthétique, homogène et durable dans le temps. En revanche les mèches de cheveux traités par la composition 5' en l'absence de chaleur i.e. à température ambiante (25 °C) ne présentent aucune modification de leur forme.

### EXEMPLES 8 et 9

### Exemple 8

Les compositions suivantes ont été préparées (en % massique) comme suit :

| Ingrédients | Invention Composition **7'** | Comparatif Composition **8'** | Témoin Composition **9'** |
|---|---|---|---|
| Propylene glycol | 10 | 10 | 10 |
| Hydroxyéthyl cellulose (PM 720 000) | 1,5 | 1,5 | 1,5 |
| Acide 1,2-dithiolane-4-méthyl-4- carboxylique | 3,2 | - | - |
| Acide lipoïque | - | 4,0 | - |
| Ammoniaque (en solution aq. à 25% en ammoniac) | qsp pH 10 | qsp pH 10 | qsp pH 10 |
| Eau distillée | qsp 100 | qsp 100 | qsp 100 |

| Ingrédients | **Composition oxydante** |
|---|---|
| Peroxyde d'hydrogène | 6 |
| Pentasodium pentétate | 0,5 |
| Eau distillée | qsp 100 |

La composition 7' correspond à une composition selon l'invention. La composition 8' est une composition, comparative, qui contient l'acide lipoïque. La composition 9' qui correspond au témoin ne contient pas de composé hétérocyclique disulfure.

Chacune des compositions 7' à 9' a été mélangée poids pour poids avec la composition oxydante, puis appliquée sur des mèches de cheveux 90% caucasiens blancs naturels dans un rapport de bain de 2, pendant 30 minutes à température ambiante.

A l'issue du temps de pause, les cheveux sont shampooinés, rincés puis séchés à 40 °C. La même opération est réalisée au total 5 fois.

L'acide cystéique (marqueur de l'intégrité de la fibre) à la surface du cheveu a été mesuré par Infra-Rouge par Transformée de Fourier (Thermo Nicolet AVATAR 330 FT-IR). Les conditions de l'analyse sont décrites de la façon suivante :
- Scanning = 64 scans
- Résolution: 4cm⁻¹
- Nombres d'onde mesurés : 800-2000 cm⁻¹
- Mode: absorbance
- Nombre de mesures par traitements : 15
- La quantité d'acide cystéique est estimée par le rapport de la hauteur au nombre d'onde de la fonction amide (1654 cm⁻¹) sur la hauteur au nombre d'onde de la fonction acide cystéique (1043 cm⁻¹).

Plus la quantité d'acide cystéique est faible moins il y a de dégradation à la surface des fibres.

Les valeurs d'acide cystéique de surface suivantes ont été obtenues :

**Tableau 1 :**

| **Composition** | **Acide cystéique de surface** |
|---|---|
| **7'** (invention) | 0,036 |
| **8'** (comparatif : état de la technique) | 0,050 |
| **9'** (témoin) | 0,059 |

D'après le tableau 1, il apparaît que l'application sur les fibres kératiniques des compositions 8' et 9' du comparatif et du témoin respectivement occasionne significativement plus de dégradation desdites fibres que lorsque les mêmes fibres sont traitées avec la composition 7' selon l'invention.

### Exemple 9

Les compositions suivantes ont été préparées (en % massique) :

| **Ingrédients** | Invention Composition **10'** | Invention Composition **11'** | Comparatif Composition **12'** | Témoin Composition **13'** |
|---|---|---|---|---|
| Propylène glycol | 10 | 10 | 10 | 10 |
| Hydroxyéthyl cellulose (PM 720 000) | 1,5 | 1,5 | 1,5 | 1,5 |
| 4,5-dihydroxy-1,2-dithiane | 3,0 | | - | - |
| 3H-1,2-benzodithian-3-one | - | 3,3 | | - |
| Acide lipoïque | - | - | 4,0 | - |
| Ammoniaque (en solution aq. à 26,6% en ammoniac) | qsp pH 10 | qsp pH 10 | qsp pH 10 | qsp pH 10 |
| Eau distillée | qsp 100 | qsp 100 | qsp 100 | qsp 100 |

| **Ingrédients** | **Composition oxydante** |
|---|---|
| Peroxyde d'hydrogène | 6 |
| Pentasodium pentétate | 0,5 |
| Eau distillée | qsp 100 |

Chacune des compositions 10' à 13' a été mélangée poids pour poids avec la composition oxydante, puis appliquée sur des mèches de cheveux 90% caucasiens blancs naturels dans un rapport de bain de 2, pendant 30 minutes à température ambiante.

A l'issue du temps de pose, les cheveux sont shampooinés, rincés puis séchés à 40 °C. La même opération est réalisée au total 5 fois.

Le gonflement diamétral des cheveux dans l'eau a été mesuré pour chacune des mèches traitées (micromètre Mitutoyo VL-50A).

Dans cette méthode, plus le diamètre du cheveu augmente, plus la reprise en eau est importante, preuve d'une altération du cheveu. La vitesse de gonflement est également calculée.

Valeurs de gonflement maximal et de vitesse de gonflement :

| | Gonflement diamétral maximal (%) | Vitesse de gonflement (% / min) |
|---|---|---|
| Cheveu naturel | 9,1 ± 1,6 | 5,3 ± 2,4 |
| Composition **10'** (invention) | 11,0 ± 1,6 | 24,3 ± 3,0 |
| Composition **11'** (invention) | 10,4 ± 1,8 | 23,6 ± 5,8 |
| Composition **12'** (comparatif) | 13,1 ± 2,5 | 28,8 ± 6,3 |
| Composition **13'** (témoin) | 12,6 ± 2,3 | 27,9 ± 7,6 |

Que ce soit sur le paramètre « gonflement maximal » que sur le paramètre « vitesse de gonflement », les valeurs obtenues à partir des fibres kératiniques traitées à l'aide des compositions selon l'invention (compositions 10', 11') indiquent une protection significative de la fibre lors du processus de traitement par rapport aux fibres traitées avec le comparatif ie avec la composition comprenant l'acide lipoïque et la composition témoin ne comprenant aucun composé hétérocyclique disulfure (compositions 12' et 13' respectivement).

## Revendications

1. Utilisation en tant qu'agent de traitement des fibres kératiniques, tels que les cheveux :
i) d'un ou plusieurs composé(s) hétérocyclique(s) disulfure(s) de formule **(I)** : 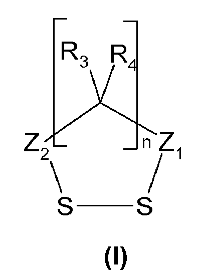 leurs sels avec un acide ou une base, leurs stéréoisomères, et leurs solvates tels que les hydrates,
formule **(I)** dans laquelle :
■ **Z₁** et **Z₂,** identiques ou différents, représentent un groupe carbonyle ou une groupe divalent CR₁R₂ ou CR₅R₆;
■ **R₁** à **R₆,** identiques ou différents, représentent un atome d'hydrogène, ou un groupement choisi parmi : i) (C₁-C₈)alkyle, ii) aryle, iii) hydroxy, iv) (di)(C₁-C₈)(alkyl)amino, v) (C₁-C₈)alkoxy, vi) (poly)hydroxy(C₁-C₈)alkyle, vii) (di)(C₁-C₈)(alkyl)amino(C₁-C₈)alkyle, viii) carboxy, ix) carboxy(C₁-C₈)alkyle, x) (di)(C₁-C₈)(alkyl)aminocarbonyl(C₁-C₈)alkyle, xii) amino, xiii) aminocarbonyle(C₁-C₈)alkyle et xiv) (C₁-C₈)(alkyl)carbonyl(C₁-C₈)(alkyl)amino(C₁-C₈)alkyle ; ou alors
■ **R₁** et **R₃,** ou lorsque n vaut 2 ou 3 deux radicaux **R₃** portés par des atomes de carbone adjacents, forment ensemble avec les atomes de carbone qui les portent un groupe cycloalkyle, hétérocycloalkyle, aryle tel que phényle, ou hétéroaryle ; particulièrement phényle ;
■ n représente un entier égal à 1, 2 ou 3, particulièrement 1 ou 2 ;
étant entendu que lorsque n vaut 2 ou 3 alors les groupement **R₃** et **R₄** peuvent être identiques ou différents entre eux ;
le(s) dit(s) composé(s) hétérocyclique(s) disulfure(s) de formule **(I)** étant associé(s) à :
ii) un ou plusieurs agent(s) réducteur(s) et/ou,
iii) en présence de chaleur apportée par chauffage à une température comprise entre 50 et 250 °C, et/ou
iv) à pH alcalin ;
étant entendu que lorsque le composé ou les composé(s) hétérocyclique(s) disulfure(s) de formule **(I)** est l'acide lipoïque et qu'il est associés à un ou plusieurs agent(s) réducteur(s), alors le dit acide lipoïque ne se trouve pas ensemble dans la même composition avec le ou les agent(s) réducteur(s).

2. Utilisation selon la revendication précédente dans laquelle le ou les composé(s) hétérocyclique(s) disulfure(s) est(sont) de formule **(I')** : 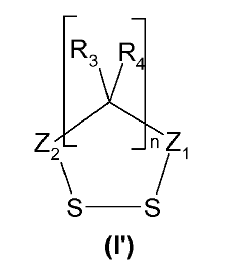 leurs sels avec un acide ou une base, leurs stéréoisomères, et leurs solvates tels que les hydrates,
formule **(I')** dans laquelle :
■ **Z₁** et **Z₂,** identiques ou différents, représentent un groupe carbonyle ou une groupe divalent CR₁R₂ ou CR₅R₆ ;
■ **R₁, R₂, R₅** et **R₆,** identiques ou différents, représentent un atome d'hydrogène, ou un groupement choisi parmi : i) (C₁-C₈)alkyle, ii) aryle, iii) hydroxy, iv) (di)(C₁-C₈)(alkyl)amino, v) (C₁-C₈)alkoxy, vi) (poly)hydroxy(C₁-C₈)alkyle, vii) (di)(C₁-C₈)(alkyl)amino(C₁-C₈)alkyle, viii) carboxy, ix) carboxy(C₁-C₃)alkyle, x) (di)(C₁-C₈)(alkyl)aminocarbonyl(C₁-C₈)alkyle, et xii) (C₁-C₈)(alkyl)carbonyl(C₁-C₈)(alkyl)amino(C₁-C₈)alkyle ;
■ **R₃** et **R₄,** identiques ou différents, représentent un atome d'hydrogène, ou un groupement choisi parmi : i) (C₁-C₈)alkyle, ii) aryle, iii) hydroxy, iv) (di)(C₁-C₈)(alkyl)amino, v) (C₁-C₈)alkoxy, vi) (poly)hydroxy(C₁-C₈)alkyle, vii) (di)(C₁-C₈)(alkyl)amino(C₁-C₈)alkyle, viii) carboxy, ix) carboxy(C₁-C₈)alkyle, x) (di)(C₁-C₈)(alkyl)aminocarbonyl(C₁-C₈)alkyle, et xii) (C₁-C₈)(alkyl)carbonyl(C₁-C₈)(alkyl)amino(C₁-C₈)alkyle ; ou alors
■ **R₁** et **R₃,** ou lorsque n vaut 2 ou 3 deux radicaux **R₃** portés par des atomes de carbone adjacents, forment ensemble avec les atomes de carbone qui les portent un groupe cycloalkyle, hétérocycloalkyle, aryle tel que phényle, ou hétéroaryle ; particulièrement phényle ;
■ n représente un entier égal à 1, 2 ou 3, particulièrement 1 ou 2 ;
étant entendu que lorsque n vaut 2 ou 3 alors les groupement **R₃** et **R₄** peuvent être identiques ou différents entre eux ; et

3. Utilisation selon une quelconque des revendications précédentes dans laquelle le ou les composé(s) hétérocyclique(s) disulfure(s) de formule **(I)** ou **(I')** sont tels que n vaut 1.

4. Utilisation selon une quelconque des revendications précédentes dans laquelle le ou les composé(s) hétérocyclique(s) disulfure(s) de formule **(I)** sont tels que n vaut 2.

5. Utilisation selon une quelconque des revendications précédentes dans laquelle le ou les composé(s) hétérocyclique(s) disulfure(s) de formule **(I)** sont tels que R₁ à R₆, identiques ou différents, représentent un atome d'hydrogène, ou un groupement choisi parmi : i) (C₁-C₈)alkyle, ii) aryle, iii) hydroxy, iv) (di)(C₁-C₈)(alkyl)amino, v) (C₁-C₈)alkoxy, vi) (poly)hydroxy(C₁-C₈)alkyle, vii) (di)(C₁-C₈)(alkyl)amino(C₁-C₈)alkyle, viii) carboxy, ix) amino, et x) (C₁-C₈)(alkyl)carbonyl(C₁-C₈)(alkyl)amino(C₁-C₈)alkyle.

6. Utilisation selon une quelconque des revendications précédentes dans laquelle le ou les composé(s) hétérocyclique(s) disulfure(s) de formule **(I)** sont tels que Z₁ représente CR₁R₂ et Z₂ représente CR₅R₆ avec R₁, R₂, R₅ et R₆ choisis parmi l'atome d'hydrogène, et les groupements hydroxy, carboxy et aminocarbonyl(C₁-C₃)alkyle.

7. Utilisation selon une quelconque des revendications précédentes dans laquelle le ou les composé(s) hétérocyclique(s) disulfure(s) de formule **(I)** ou **(I')** sont tels que R₃ et R₄, identiques ou différents, représentent un atome d'hydrogène ou un radical choisi parmi (C₁-C₃)alkyle, hydroxy et carboxy.

8. Utilisation selon une quelconque des revendications précédentes dans laquelle le ou les composés hétérocycliques de formule **(I)** est(sont) tel(s) que Z₁ et/ou Z₂ désigne(nt) un groupe carbonyle, et/ou R₁ et R₃ ou deux radicaux R₃ portés par des atomes de carbone adjacents forment ensemble avec les atomes de carbone qui les portent un cycle benzénique.

9. Utilisation selon une quelconque des revendications précédentes dans laquelle le ou les composé(s) hétérocyclique(s) disulfure(s) de formule **(I)** est(sont) choisi(s) parmi les composés (1) à (6), leurs stéréoisomères et leurs sels d'addition avec un acide ou une base ou leurs hydrates:
| **Dénomination** | **Formule chimique** |
|---|---|
| Acide lipoïque | 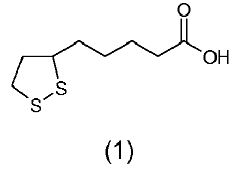 |
| Lipoamide | 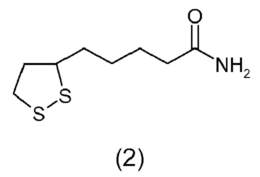 |
| Acide asparagusique | 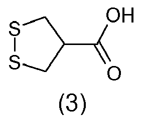 |
| 4,5-dihydroxy-1,2-dithiane | 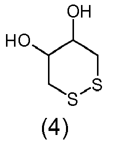 |
| 3H-1,2-benzodithiol-3-one | 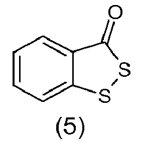 |
| Acide 1,2-dithiolane-4-méthyl-4- carboxylique | 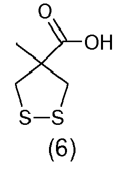 |

10. Utilisation selon une quelconque des revendications précédentes dans laquelle dans leurs formes cycliques le ou les composé(s) hétérocyclique(s) de formule **(I)** présente(nt) un logP inférieur à 3.

11. Utilisation selon une quelconque des revendications précédentes dans laquelle le ou les composé(s) hétérocyclique(s) de formule **(I)** ou leurs formes réduites est(sont) présent(s) dans une composition à une concentration comprise entre 1 et 20 % en poids par rapport au poids total de la composition les contenant.

12. Utilisation selon une quelconque des revendications précédentes dans laquelle le ou les composé(s) hétérocyclique(s) de formule **(I)** ou leurs formes réduites est(sont) présent(s) dans une composition qui comprend un ou plusieurs agent(s) alcalinisant(s) tel(s) que :
a) l'ammoniaque,
b) les alcanolamines telles que les mono-, di- et triéthanolamines ainsi que leurs dérivés,
c) les éthylènediamines oxyéthylénées et/ou oxypropylénées,
d) les hydroxydes minéraux ou organiques,
e) les silicates de métaux alcalins,
f) les acides aminés de préférences basiques,
g) les (bi)carbonates particulièrement d'amine primaire, secondaire ou tertiaire (ammonium), de métal alcalin ou alcalino-terreux et
h) les composés de formule (II) suivante : 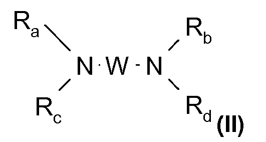
formule **(II)** dans laquelle W est un reste propylène éventuellement substitué par un groupement hydroxy ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

13. Utilisation selon une quelconque des revendications précédentes dans laquelle le ou les agent(s) réducteur(s) est(sont) choisi(s) parmi l'acide thioglycolique et ses sels, le monothioglycolate de glycérol ou de glycol, la cystéamine et ses dérivés acylés en C₁-C₄, la cystéine, la N-acétylcystéine, les esters de cystéine, les N-mercaptoalkylamides de sucres, l'acide thiolactique et ses esters, l'acide 3-mercaptopropionique et ses esters, l'acide thiomalique, l'acide 2-hydroxy 3-mercaptopropionique et ses esters, la panthétéine, le thioglycérol, les N-(mercaptoalkyl)ω -hydroxyalkylamides et les N-mono- ou N,N-dialkylmercapto 4-butyramides, les aminomercaptoalkylamides, les dérivés des acides N-(mercaptoalkyl) succinamiques ou des N-(mercaptoalkyl) succiminides, les alkylaminomercaptoalkylamides, le mélange de thioglycolate d'hydroxy-2-propyle et de thioglycolate d'hydroxy-2-méthyl-1 éthyle et les N-mercaptoalkyl alcanediamides.

14. Utilisation selon une quelconque des revendications précédentes dans laquelle le ou les composé(s) hétérocyclique(s) de formule **(I)** ou leurs formes réduites est(sont) présent(s) dans une composition qui comprend un ou plusieurs agent(s) réducteur(s) tels que définis dans la revendication 1 ou 13 présents en quantité comprise entre 0,01 et 20 % en poids par rapport au poids de la composition les comprenant.

15. Utilisation selon une quelconque des revendications précédentes en tant qu'agent de déformation permanente des fibres kératiniques tels que les cheveux ou lorsque l'utilisation est réalisée du ou des composé(s) hétérocyclique(s) disulfure(s) iv) à pH alcalin pour diminuer la dégradation des fibres kératiniques.

16. Composition cosmétique à pH alcalin comprenant :
i) un ou plusieurs composé(s) hétérocycliques disulfures de formule **(I)** ou leurs formes réduites tels que définis dans une quelconque des revendications 1 à 11.

17. Composition cosmétique comprenant :
i) un ou plusieurs composé(s) hétérocyclique(s) disulfure(s) de formule **(I')** tels que défini dans une quelconque des revendications 2 à 11 et
ii) un ou plusieurs agent(s) réducteur(s) tels que définis dans la revendication 1, 13 ou 14.

18. Composition cosmétique selon les revendications 16 et 17 qui est à pH alcalin et contient un ou plusieurs agent(s) alcalinisant(s) tel(s) que défini(s) dans la revendication 12.

19. Composition selon une quelconque des revendications 16 à 18 qui contient une ou plusieurs bases d'oxydation et éventuellement un ou plusieurs coupleur(s).

20. Composition selon une quelconque des revendications 16 à 19 qui contient un ou plusieurs colorants directs.

21. Composition selon une quelconque des revendications 16 à 20 qui contient un ou plusieurs agents oxydants.

22. Procédé de traitement des fibres kératiniques, par application sur les fibres kératiniques humaines d'une composition telle que définie dans une quelconque des revendications 16 à 21.

23. Procédé de traitement des fibres kératiniques, selon la revendication précédente qui comprend :
- une première étape dans laquelle on applique sur les fibres kératiniques à traiter une composition telle que définie dans une quelconque des revendications 16 à 21 ;
- une deuxième étape dans laquelle on laisse pendant un temps de pause de 1 minute à 2 heures la composition prête à l'emploi ;
- une troisième étape dans laquelle les fibres kératiniques sont shampooinées, rincées et éventuellement séchées.

24. Procédé de traitement des fibres kératiniques en vue d'obtenir une déformation permanente de ces dernières, par application successive sur les fibres kératiniques à traiter :
- dans un premier temps d'un ou plusieurs agent(s) réducteur(s) tels que définis dans une quelconque des revendications 1, 13 ou 14 puis
- dans un deuxième temps d'un ou plusieurs composé(s) hétérocyclique(s) disulfure(s) de formule **(I)** tels que définis dans une quelconque des revendications 1 à 11.

25. Procédé de traitement des fibres kératiniques, selon la revendication précédente qui comprend :
- une première étape dans laquelle on applique sur les fibres kératiniques à traiter un ou plusieurs agent(s) réducteur(s) tels que définis dans une quelconque des revendications 1, 13 ou 14 ;
- une deuxième étape dans laquelle on rince lesdites fibres ;
- une troisième étape dans laquelle on applique sur lesdites fibres un ou plusieurs composé(s) hétérocyclique(s) disulfure(s) de formule **(I)** tels que définis dans une quelconque des revendications 1 à 11.

26. Procédé selon une quelconque des revendications 22 à 25 qui est suivie d'une étape de fixation des fibres kératiniques à l'aide d'un ou plusieurs agent(s) oxydant(s) puis éventuellement d'une étape de rinçage.

27. Procédé selon une quelconque des revendications 22 à 26 en présence de chaleur apportée par chauffage à une température comprise entre 50 et 250 °C, particulièrement entre 50 °C et 100 °C.

28. Procédé pour le traitement des fibres kératiniques en vue d'obtenir une déformation permanente de ces dernières dans lequel lors de la première étape du procédé le ou les composé(s) hétérocyclique(s) disulfure(s) de formule (I) tels que définis dans une quelconque des revendications 1 à 11 sont appliqués sur les fibres kératiniques et dans une deuxième étape les fibres kératiniques sont chauffées à une température comprise entre 50 et 250 °C, particulièrement entre 50 °C et 100 °C.
